# EUROPÄISCHE PATENTSCHRIFT

(11) **EP 1 325 334 B1**
(45) Veröffentlichungstag und Bekanntmachung des Hinweises auf die Patenterteilung: **30.11.2011**
(21) Anmeldenummer: 01980432.7
(22) Anmeldetag: 25.09.2001
(51) Int. Cl.: G01N 33/543

(54) **VERFAHREN ZUR IMMOBILISIERUNG VON LIPIDSCHICHTEN**
METHOD FOR IMMOBILISING LIPID LAYERS
PROCEDE POUR L'IMMOBILISATION DE COUCHES LIPIDIQUES

(30) Priorität: 29.09.2000 DE 10048822
(43) Veröffentlichungstag der Anmeldung: 09.07.2003
(73) Patentinhaber: Nimbus Biotechnologie GmbH, 04317 Leipzig (DE)
(72) Erfinder: NÖLLER, Joachim, 04105 Leipzig (DE); SCHMITT, Johannes, 04157 Leipzig (DE)
(74) Vertreter: Patentanwälte Ruff, Wilhelm, Beier, Dauster & Partner
(86) Internationale Anmeldenummer: PCT/EP2001/011036
(87) Internationale Veröffentlichungsnummer: WO 2002/027320

(56) Entgegenhaltungen:
- EP-A- 0 930 363
- DE-A- 4 128 953
- FR-A- 2 751 989
- LINDHOL-SETHSON ET AL: "Electron transfer to a gold electrode from cytochrome oxidase in a biomembrane via a polyelectrolyte film" LANGMUIR, Bd. 14, Nr. 6, 1998, Seiten 6705-6708, XP002196775

## Beschreibung

Die Erfindung betrifft ein Verfahren zur Immobilisierung von Lipiddoppelschichten auf Oberflächen partikelförmiger Substrate, so dass die Substratoberfläche von der Lipiddoppelschicht vollständig umschlossen wird, sowie gemäß dem Verfahren herstellbare partikelförmige Substrate.

Festkörperunterstützte Lipiddoppelschichten (im folgenden Bilayer genannt) und festkörperunterstützte funktionelle Biomembranen mit darin immobilisierten Membranproteinen haben in den letzten Jahren im Bereich des Pharmascreenings, der Chromatographie, der Biosensorik, und für Lipidbindungsstudien an Bedeutung gewonnen. Die ersten in der Literatur beschriebenen festkörperunterstützten Bilayer waren nur durch einen 1 bis 2 nm dicken Wasserfilm von der Festkörperoberfläche entfernt (Tamm, L. K.; McConnell, H. M. Biophysical Journal 1985, 47, 105-113; Johnson, S. J.; Bayerl, T. M.; McDermott, D. C.; Adam, G. W.; Rennie, A. R.; Thomas, R. K.; Sackmann, E. Biophysical Journal 1991, 59, 289-294; Bayerl, T. M.; Bloom, M. Biophysical Journal 1990, 58, 357-362), wodurch wesentliche Bewegungseigenschaften des Bilayers (z.B. Diffusion der Lipide in der Bilayerebene (M. Hetzer, S. Heinz, S. Grage, T. M. Bayerl, Langmuir, 1998, 14, 982-984)) vom Festkörper dominiert wurden. Mit dieser Technik wurden auch in Liposomen rekonstituierte Proteine mittels Fusion auf unbehandelten, planaren Oberflächen immobilisiert (J. Salafsky, J. T. Groves, S. G. Boxer, Biochemistry, 1996, 35, 14773 - 14781). Die daraus resultierenden Unterschiede zu natürlichen Zellmembranen sowie der geringe Abstand zwischen Bilayer und Festkörper erschwerten insbesondere die funktionelle Immobilisierung von integralen Membranproteinen. So beschäftigen sich neuere Forschungsarbeiten insbesondere mit einer Erhöhung des Abstandes zwischen Bilayer und Festkörper, um eine dynamische Entkopplung des Bilayers zu erreichen und integralen Membranproteinen im Bilayer einen ausreichend großen Abstand zur häufig denaturierend wirkenden Festkörperoberfläche zu ermöglichen. Dies ist beispielsweise eine Voraussetzung für die Verwendung von lonenkanälen im Bilayer als Biosensor.

Aktuelle Strategien zur Erreichung dieses Ziels bestehen insbesondere darin, Lipidmoleküle über hydrophile Spacer ("Abstandshalter") auf der Festkörperoberfläche chemisch oder physikalisch anzubinden (B. A. Cornell, V. L. Braach-Maksvytis, L. G. King, P. D. Osman, B. Raguse, L. Wieczorek, R. J. Pace, Nature, 1997, 387, 580; S. Lingler, I. Rubinstein, W. Knoll, A. Offenhäuser, Langmuir, 1997, 13, 7085; H. J.Galla, C. Steinem, K. Rheis, Patent DE 19607279), wobei der hydrophile Spacer zur Entkopplung des aus weiteren Lipidmolekülen gebildeten Bilayers vom Substrat dient, indem die gebundenen Lipide als "Anker" wirken und damit die maximale Entfernung zwischen Bilayer und Oberfläche durch die Länge des hydrophilen Spacers vorgeben. Dabei können die Lipidmoleküle entweder an polymere, oligomere oder niedermolekulare Spacer gebunden sein. Nachteilig ist der hohe präparative Aufwand bei diesen Strategien und die Abhängigkeit der Eigenschaften des Bilayers von der lateralen Dichte der Ankermoleküle, von der Hydratisierbarkeit des hydrophilen Spacermaterials sowie von den dynamischen Eigenschaften des Spacers selbst.

Einen weiteren Schritt zu einem weitgehend von der Festkörperoberfläche entkoppelten Bilayer stellt deshalb die Verwendung von Festkörperoberflächen dar, die durch ein hydrophiles Polymer (z.B. Dextran) modifiziert und anschließend mit einem Bilayer beschichtet wurden (M. Kuhner, E. Sackmann, Langmuir, 1996 12 (20), 4866; G. Elender, M. Kuhner, E. Sackmann, Biosens. Bioelectron., 1996, 11, 565; E. Sackmann, Science, 1996, 271, 43; E. Györvary, B. Wetzer, U.B. Sieytr, A. Sinner, A. Offenhäusser, W. Knoll, Langmuir 1999, 15, 1337). In diesem Zusammenhang sind auch die im folgenden beschriebenen Arbeiten zu sehen, die als hydrophile Polymere Polyelektrolytmono- oder -multilagen verwenden, um den Bilayer von einer planaren Festkörperoberfläche zu entkoppeln. In (J. Majewski, J.Y. Wong, C.K. Park, M. Seitz, J.N. Israelachvili, G.S. Smith, Biophysical Journal 1998, 75, 2363) ist die Möglichkeit beschrieben, eine bereits bestehende festkörperunterstützte Lipidmembran auf einem planaren Quarzsubstrat durch Behandlung mit einem verzweigten kationischen Polyelektrolyten nachträglich von der Festkörperoberfläche zu entkoppeln, wobei allerdings ein schrittweiser Aufbau bzw. eine direkte Fusion der Vesikel auf der Polykation-Lage nicht zu einem definierten Bilayer führte. In (J.Y. Wong, J. Majewski, M. Seitz, C.K. Park, J.N. Israelachvili, G.S. Smith, Biophysical Journal 1999, 77, 1445) wird gezeigt, daβ eine definierte Lipidmembran nur auf einer vorher getrockneten PEI-(Polyethylenimin) Lage erhalten werden kann. Dagegen gelang (U. Sohling, A.J. Schouten, Langmuir 1996, 12, 3912) der Aufbau einer festkörperunterstützten Lipidmembran auf PEI-modifizierten Oberflächen im Falle von negativ geladenen Lipiden. In (B. Lindholm-Sethson, Langmuir 1996, 12, 3305) ist beschrieben, daß definierte Bilayer aus partiell negativ geladenen Lipiden ebenfalls auf anionischen PSS (Na-Polystyrolsulfonat) Oberflächen durch Verwendung von Ca²⁺-Brücken erhalten werden. In (B. Lindholm-Sethson, J.C. Gonzales, G. Puu, Langmuir 1998, 14, 6705) wird schließlich gezeigt, daβ Cytochrom C-Oxidase enthaltende Proteoliposomen auf Polyelektrolytmultilagen funktionell immobilisiert werden können.

Alle diese Arbeiten wurden jedoch auf planaren Substraten vorgenommen, wodurch nur eine geringe Gesamtoberfläche zur Verfügung steht, was beispielsweise chromatographische Anwendungen unmöglich macht, und andere Anwendungen erschwert.

Weitere Ansätze zur Herstellung polymerunterstützter Lipidmembranen nutzen sogenannte Filmwaagentechniken wie Langmuir-Blodgett und Langmuir-Schäfer, und sind daher im wesentlichen beschränkt auf planare Oberflächen, und ebenfalls nicht geeignet für bestimmte Anwendungen. Ein Beispiel hierfür ist beschrieben in (H. Hillebrandt, G. Wiegand, M. Tanaka, E. Sackmann, Langmuir 1999, 15, 8451).

Aus der DE 4128953 A1 ist ein Verfahren zur Zellkultivierung bekannt, bei dem Säugerzellen auf die Oberflächen von Glaskügelchen aufgebracht werden. Die Zellen bilden jedoch keine die Oberfläche der Glaskügelchen umschließende Lipiddoppelschicht aus.

Der Verzicht auf Spacerelemente ermöglicht dem Bilayer bei dieser Strategie eine höchstmögliche Entkopplung von der Festkörperoberfläche und gleichzeitig können auf diese Weise integrale Proteine in die Membran eingebettet werden ohne direkt in Kontakt mit dem Festkörper zu gelangen. Das entscheidende Problem dieser Methode ist die Stabilität des Bilayers auf der Polymeroberfläche, die ein wichtiges Kriterium für Anwendungen in der Bioanalytik und Biosensorik ist. Externe Kräfte (z.B. Flußkräfte eines vorbeiströmenden Mediums) führen bei dieser Strategie daher leicht zur Ablösung von Teilen des Bilayers. Weitere Strategien beruhen auf der Ausbildung von Lipidmonolagen (im folgenden als Monolayer bezeichnet) auf Festkörperoberflächen, die zuvor mittels Alkylsilan- oder Mercaptanmonolagen hydrophobisiert wurden. Der Nachteil ist hier offensichtlich: ein reales Entkoppeln von der Substratoberfläche findet nicht statt, was die Dynamik und molekulare Ordnung der Monolayer signifikant gegenüber denen eines Bilayers verändert (Linseisen, F. M.; Hetzer, M.; Brumm, T.; Bayerl, T. M. Biophysical Journal 1997, 72, 1659-1667) und den Einbau von transmembranen Proteinen erschwert oder gar verhindert. So zeigten C. Steinem et al. (Steinem, C.; Janshoff, A.; Galla, H.J.; Sieber, M., Bioelectrochem. Bioenerg. 1997, 42 (2), 213-220), daß eine Fusion der Proteoliposomen zum Lipidbilayer wahrscheinlich nicht stattfindet, sondern nur eine Anlagerung bzw. Teilfusion, so daß letztlich immobilisierte Vesikel auf der Oberfläche vorliegen. Ältere Arbeiten zu diesem Thema untersuchten diese Möglichkeit nicht bzw. ihre gezeigten funktionellen Daten lassen ebenso die Interpretation zu, daß kein direkter Proteineinbau in einem Lipidbilayer stattfindet und die Enzymaktivität aus den vesikulären Strukturen resultiert.

Die Erfindung stellt sich nun die Aufgabe, ein weitgehend universell anwendbares Verfahren zur Verfügung zu stellen, welches die beschriebenen Mängel der herkömmlichen Strategien zur Immobilisierung von Lipidschichten vermeidet und auf eine präparativ einfache Art die Entkopplung der Lipidschichten, insbesondere der Lipiddoppelschichten, von der Membran erlaubt. Bei dem neuen Verfahren soll die Festkörperoberffäche derart optimiert werden, daß die Eigenschaften der festkörperunterstützten Lipidschichten bei hoher Stabilität gegen äußere Kräfte möglichst nahe den Eigenschaften natürlicher Membransysteme sind.

Diese Aufgabe wird gelöst durch das Verfahren mit den Merkmalen des Anspruchs 1. Bevorzugte Ausführungsformen des Verfahrens sind in den Ansprüche 2 bis 22 dargestellt. Anspruch 23 betrifft ein gemäß dem erfindungsgemäßen Verfahren herstellbares partikelförmiges Substrat.

Bei dem erfindungsgemäßen Verfahren werden Festkörperoberflächen zunächst derart modifiziert, daß diese optimale Bedingungen für die schonende Immobilisierung einer über der modifizierten Oberfläche aufgebrachten Lipiddoppelschicht bzw. Lipidmembran bieten. In einem zweiten Schritt des Verfahrens werden sodann Lipidschichten, beispielsweise Membranen aus nativen Zellen, so auf der modifizierten Oberfläche immobilisiert, daß die Eigenschaften der immobilisierten Lipidschichten im wesentlichen denen nicht-immobilisierter Lipidschichten entsprechen. Dabei erfolgt die Immobilisierung der Lipidschichten mittels Fusion von Vesikeln aus Pufferlösungen.

Bei den genannten Eigenschaften der Lipidschichten handelt es sich zunächst um die Diffusivität der Lipidschicht. Eine nicht-immobilisierte Lipidschicht, insbesondere eine Lipiddoppelschicht, zeichnet sich dadurch aus, daß die einzelnen Lipide und andere Komponenten, beispielsweise Proteine, innerhalb der Schicht und bei einer Doppelschicht auch zwischen den beiden Schichten relativ frei beweglich sind. Bei herkömmlichen Immobilisierungsmethoden wird diese Diffusivität der Lipidschichten extrem herabgesetzt. Bei einer Doppelschicht wird vor allem in der Lipidlage, die der Oberfläche des Substrates zugeordnet ist, die Bewegungsmöglichkeit der Lipide deutlich abgebremst. Dies führt zu einer drastischen Veränderung der Eigenschaften der herkömmlich immobilisierten Membran. Bei der erfindungsgemäßen Immobilisierungsmethode wird die Bewegungsmöglichkeit innerhalb der Lipidschicht nicht eingeschränkt, so daß diese charakteristische Eigenschaft bei den erfindungsgemäß immobilisierten Lipidschichten erhalten bleibt.

Weiterhin wird durch die erfindungsgemäße Immobilisierungsmethode durch die Modifizierung der Festkörperoberfläche eine "weiche" Oberfläche erzielt, die zum einen die darauf immobilisierte Lipidschicht weitgehend unbeeinflußt läßt und zum anderen auch einen ausreichenden Abstand zwischen Festkörperoberfläche und Lipidschicht gewährleistet, so daß keine denaturierenden Effekte der Festkörperoberfläche auf die evtl. in der Lipidschicht enthaltenden Proteine, insbesondere Enzyme, auftreten. Hierdurch wird erreicht, daß Aktivitäten von in der Lipidschicht eingelagerten Proteinen erhalten bleiben.

Dieses Merkmal der Erfindung ist ausgesprochen wichtig für die verschiedenen Anwendungsmöglichkeiten der Erfindung. Vor allem in der Biosensorik sind immobilisierte Membranen, die ihre nativen Eigenschaften, also beispielsweise enzymatische Aktivitäten oder auch Kanalaktivitäten, beibehalten, ausgesprochen wichtig. Mit herkömmlichen Methoden war eine derartige Immobilisierung von Membranen bei Erhalt der nativen Eigenschaften nicht möglich. Durch die Erfindung erschließen sich daher ganz neue Möglichkeiten in der Biosensorik und natürlich auch in ganz verschiedenen anderen Bereichen, wie beispielsweise in der Diagnostik oder in der Forschung allgemein.

Nach dem bisherigen Wissenstand war es nicht vorstellbar, daß solche "dynamisch" immobilisierten Membranen eine ausreichende Stabilität auf dem jeweiligen Träger aufweisen würden. Vielmehr war der Fachmann davon ausgegangen, daß für eine Immobilisierung eine konkrete Fixierung der Membran, insbesondere durch die bereits oben erwähnten Linker, notwendig sei, damit die Membran sich nicht sofort vom Träger wieder ablöst. Die andere bekannte Möglichkeit für eine ausreichend stabil immobilisierte Lipidschicht war ein relativ geringer Abstand der Lipidschicht zum Träger, wie er durch eine oben erwähnte Immobilisierung mit Hilfe von Dextran oder Polyelektrolyten erreicht wird. Der Fachmann war folglich davon ausgegangen, daß eine immobilisierte Membran entweder kovalent fixiert sein muß - mit dem Nachteil der fehlenden Diffusivität innerhalb der Membran - oder daß die Membran sehr nah an den Träger gebracht werden muß, wobei allerdings der Träger denaturierende Effekte auf die Lipidschicht bzw. auf die darin enthaltenen Proteine ausübt.

Die von den Erfindern erzielten Resultate mit erfindungsgemäß modifizierten Membranen stellen daher sehr überraschende Ergebnisse dar, da es sich zeigte, daβ die "dynamisch" immobilisierten Lipidschichten derart stabil auf ihren Trägern immobilisiert sind, daß sie für alle denkbaren Anwendungen geeignet ist. Die erfindungsgemäß immobilisierten Lipidschichten weisen einen ausreichenden Abstand von dem Träger auf, so daß keine denaturierenden Effekte des Trägers auftreten. Darüberhinaus werden Linker vermieden, welche die Membran an distinkten Stellen fixieren würden. Hierdurch wird gewährleistet, daß eine erfindungsgemäß immobilisierte Lipidschicht ihre dynamischen Eigenschaften, insbesondere die Diffusivität der verschiedenen Lipidschichtkomponenten und Aktivitäten von möglichen weiteren Schichtkomponenten, beibehält.
In der nachfolgenden Beschreibung werden einige Fachbegriffe aus der Biophysik und der Oberflächen- und Polymerchemie verwendet, die hier kurz erläutert werden. Für eine ausführliche Erläuterung sei auf die Fachliteratur verwiesen (A. Ulman "An Introduction to Ultrathin Organic Films", Academic Press, Inc., 1991; Albert L. Lehninger "Prinzipien der Biochemie" Walter de Gruyter 1987; R. W. Armstrong, U. P. Strauss "Polyelectrolytes" in "Enzyclopedia of Polymer Science and Technology", Eds. A. Klingsber, R. M. Piccinne, A. Salvatore, John Wiley and Sons, 1969, Volume 10, 781; M. Mandel "Polyelectrolytes" in "Enzyclopedia of Polymer Science and Technology", Eds. N. M. Bikales, J. Conrad, A. Ruks, John Wiley and Sons, 1988, Volume 11, 739; H. G. M. van de Steg, M. A. Cohen Stuart, A. de Keizer, B. H. Bijsterbosch, Langmuir, 1992, 8, 2538).

Die im nachfolgenden Text beschriebenen "Polyionen" beschreiben allgemein Polymere, die ionische und/oder ionisierbare Funktionalitäten entweder in den Seitenketten und/oder entlang der Hauptkette tragen. Unter Polyionen sollen nachfolgend die Molekülklassen "Polyelektrolyte", "Polyampholyte" und "Polyzwitterionen" verstanden werden.

"Polyelektrolyte" sind Polymere, die ionische oder ionisierbare Gruppen in der Haupt- oder Seitenkette eingebaut haben. "Polyelektrolyte" im nachfolgend verwendeten Sinn können auch Copolymere aus ionischen/ionisierbaren und nichtionischen Monomereinheiten sein. Polyelektrolyte können sowohl in anionischer wie auch in kationischer Form vorliegen. Beispiele für anionische Polyelektrolyte sind Poly(styrolsulfonsäure), Polyvinyl(sulfonat), Poly(acrylsäure), Dextransulfat, PAMAM-Dendrimere (Poly(amidoamine), Carboxylterminierte, halbzahlige Generation) und Carboxycellulose. Komplexere Formen von anionischen Polyelektrolyten sind beispielsweise Desoxyribonukleinsäuren (DNA) und Ribonukleinsäuren (RNA). Beispiele für kationische Polyelektrolyte sind Poly(allylaminhydrochlorid), Poly(vinylamin), Poly(ethylenimin), Poly(diallylammoniumchlorid), PAMAM-Dendrimere (Aminoterminierte, ganzzahlige Generation) und Poly(2-vinylpyridin). Beispiele für ionische Copolymere sind Poly(acrylsäure-co-acrylamid) und Poly(diallylammonium-chlorid-co-acrylamid). Aus dieser kurzen Auflistung ist ersichtlich, daß typische funktionelle Gruppen anionischer Polyelektrolyte Carboxyl-, Sulfat-, Sulfonat-, Phosphat- und Phosphonatgruppen sind. Typische kationische Funktionalitäten sind primäre, sekundäre, tertiäre und quaternäre Amin-, sowie auch R₃S(+)-Gruppen.

Der Gruppe der Polyelektrolyte verwandte Polymere sind Polyampholyte, die in der Haupt- oder Seitenkette ionisierbare funktionelle Gruppen tragen, und deren Netto-Ladungszustand von dem pH-Wert der Lösung abhängt. Unter Polyampholyten im allgemeinen Sinn sollen auch Proteine und Enzyme verstanden werden.

Eine weitere Gruppe ionischer Polymere sind Polyzwitterionen, die in der Haupt- oder Seitenkette permanente anionische und kationische Ladungen tragen.

Die im nachfolgenden Text verwendeten Begriffe "Bilayer", "Lipidmembran" und "Lipidbilayer" sind synonym und beziehen sich auf eine Lipiddoppelschicht, die aus einem hydrophoben inneren Bereich und einem hydrophilen äußeren Bereich besteht, und die beispielsweise bei der Selbstorganisation von natürlichen und synthetischen Lipiden oder lipid-ähnlichen Substanzen in einer wäßrigen Phase spontan entstehen, oder durch Übertragungstechniken (Langmuir-Blodgett-Technik) erzeugt werden können.

Der Begriff "Vesikel" bezieht sich auf uni- und multilamellare Aggregatformen, die Lipide und lipidähnliche Substanzen bei Quellung in wäßriger Phase spontan, oder unter äußerem Einfluß, wie beispielsweise durch Behandlung mit Ultraschall oder durch Hochdruckfiltration (Extrusion), ausbilden.

Der im folgenden Text verwendete Begriff "Substrate" bezieht sich auf in wäßriger Lösung unlösliche Festkörper aus organischem oder anorganischem Material, die als ausreichend feste Oberflächen (Festkörperoberflächen) nach der Optimierung zur Unterstützung der Lipidschichten dienen.

Die Erfindung beschreibt ein neues Verfahren zur Optimierung der Oberflächeneigenschaften von pulver- oder partikelförmigen Substraten beliebiger Geometrie mit dem Ziel, die Eigenschaften der hierauf zu deponierenden Lipidschichten möglichst weit an jene einer natürlichen Membran anzunähern und gleichzeitig die Immobilisierung integraler Membranproteine ohne signifikanten Verlust ihrer Aktivität zu ermöglichen.

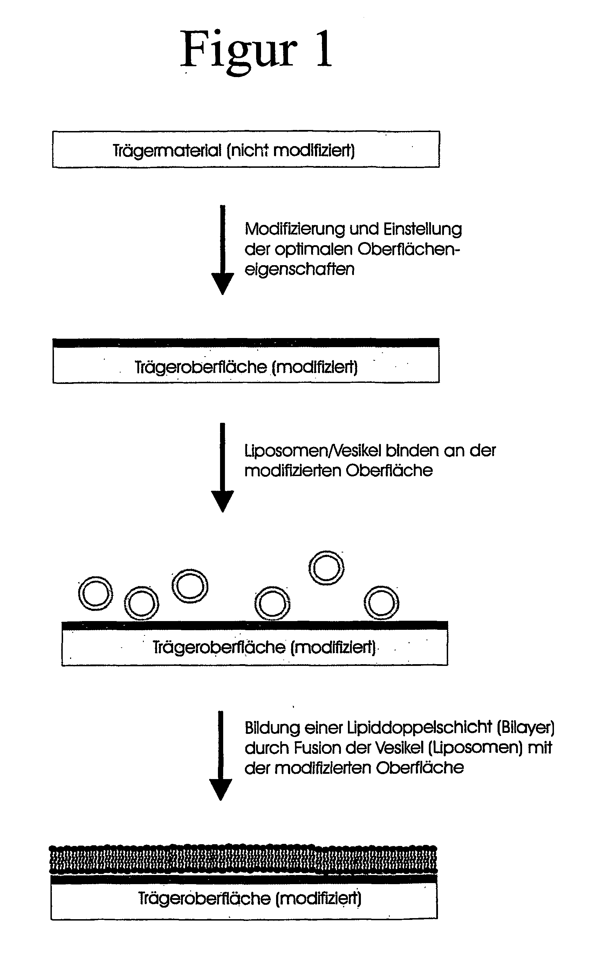

Das erfindungsgemäße Verfahren läßt sich in zwei Verfahrensschritte unterteilen. Dies ist zum einen (a) die Modifizierung der Festkörperoberfläche (Substratoberfläche) mit Molekülen zur Ausbildung einer im wesentlichen hydrophilen Fläche und (b) das Deponieren der Lipidschichten auf der modifizierten Festkörperoberfläche. Diese Verfahrensschritte sind schematisch der Figur 1 zu entnehmen, wobei der pulverförmige Charakter des Festkörpers nicht dargestellt ist. Durch den ersten Verfahrensschritt wird eine geeignete Modifizierung der Substratoberfläche erreicht, die eine weitgehende Entkopplung der im zweiten Verfahrensschritt zu deponierenden Lipidschicht vom Substrat bei gleichzeitiger hoher morphologischer Integrität und Stabilität gewährleistet. Hierbei wird die Verwendung von kovalent an das Substrat gebundenen Spacerelementen vermieden. Die immobilisierte Lipidschicht ist also frei, d.h. ohne punktuelle Verbindung an das Substrat, auf der modifizierten Oberfläche immobilisiert.

Durch die Modifizierung der Oberfläche gemäß Verfahrensschritt (a) kann insbesondere die Ausbildung einer hydrophilen Oberfläche erreicht werden, die ionische Gruppen trägt, d.h. vorzugsweise funktionelle Gruppen, die in wässriger Umgebung dissoziieren.

Darüberhinaus ist die entstehende festkörperunterstützte Lipidschicht den Eigenschaften ihres natürlichen (festkörperunabhängigen) Analogons (z.B. dem Lipidvesikel im Fall von reinen Lipiden oder der Biomembran im Fall von natürlichen Lipidmischungen mit Proteinen, insbesondere Transmembranproteinen) sehr ähnlich.

Für die Modifizierung der Festkörperoberfläche (Verfahrensschritt (a)) sind im wesentlichen zwei Schritte notwendig. Zunächst (aa) wird die Festkörperoberfläche funktionalisiert, d.h. es werden funktionelle Gruppen, insbesondere chemisch funktionelle Gruppen, auf der Oberfläche fixiert. In einem weiteren Schritt (ab) werden auf der funktionalisierten Oberfläche wechselwirkende Moleküle adsorbiertlchemisorbiert. Weiterhin können in einem dritten Schritt (ac) weitere Moleküle adsorbiert oder chemisorbiert werden. Diese Moleküle können identisch mit den in Schritt (ab) eingesetzten Molekülen sein, oder es kann eine andere Molekülspezies für diesen weiteren Schritt verwendet werden. Darüberhinaus kann die Modifizierung auch noch weitere entsprechende Teilschritte umfassen. Dies kann dann von Nutzen sein, wenn der Abstand der Lipidschicht zur Festkörperoberfläche besonders groß ausgebildet sein soll. Eine Modifizierung in wenigen Schritten ist jedoch oft auch im Hinblick auf den präparativen Aufwand besonders vorteilhaft und bevorzugt. Das Aufbringen der entsprechenden Moleküle kann beispielsweise durch Deposition aus einer Lösung oder aus der Gasphase vorgenommen werden.

Die Modifizierung der Oberfläche kann auch in einem Schritt durchgeführt werden, insbesondere wenn ein geeignetes oberflächenmodifizierendes Material kommerziell erhältlich ist.

Durch eine entsprechende Modifizierung der Festkörperoberfläche (Verfahrensschritt (a)) wird die Ausbildung einer hydrophilen Oberfläche auf dem Substrat erreicht. Hierdurch wird eine spontane Bildung einer Lipidschicht, insbesondere eines Bilayers, auf der modifizierten Oberfläche (Verfahrensschritt (b)) ermöglicht. Dies wird durch eine herkömmliche Vesikelfusion auf der Oberfläche erzielt.

Nach beiden Verfahrensschritten (a) und (b) - Modifizierung der Oberfläche und Deponierung bzw. Immobilisierung der Lipidschicht - ist das Ergebnis eine stabile festkörperunterstützte Lipidschicht, vorzugsweise ein Bilayer oder eine festkörperunterstützte Biomembran mit enzymatischer Aktivität der in ihr immobilisierten Proteine. Die Topologie der Lipidschicht bzw. Bilayer- oder Membranoberfläche ist hierbei weitgehend durch die des Substrates, also der Festkörperoberfläche, vorgegeben.

Das erfindungsgemäße Verfahren zeichnet sich vorzugsweise also dadurch aus, daß anorganische und/oder organische Festkörperoberflächen chemisch und/oder physikalisch derart modifiziert werden, daß
a) die Oberflächeneigenschaften des Trägermaterials (Substrats) in gezielter Weise beeinflußbar und einstellbar sind;
b) das Aufbringen einer Lipidschicht auf die modifizierte Festkörperoberfläche ermöglicht wird;
c) die Lipidschicht durch physikalische und/oder chemische Kräfte so an die modifizierte Festkörperoberfläche gebunden wird, daß sie diese vollständig umschließt, ohne daß jedoch die Diffusion der Lipidschichtbestandteile innerhalb der Schicht wesentlich beeinträchtigt ist;
d) die Eigenschaften der immobilisierten Lipidschicht, insbesondere einer Lipidmembran, möglichst nahe den Eigenschaften der nichtimmobilisierten Lipidschicht bzw. der natürlichen Lipidmembran sind.

Kriterien für den Vergleich der Eigenschaften der durch das erfindungsgemäße Verfahren erhaltenen immobilisierten Lipidschicht mit denen ihrer festkörperfreien Analoga sind beispielsweise Messungen der Phasenübergangstemperatur mittels Differential-Mikrokalorimetrie (DSC), Messung der Fluidität und Mobilität mittels Festkörper-Kernresonanz (NMR) oder Bestimmung der Funktionalität bzw. Aktivität immobilisierter Membranproteine.

Bei dem bevorzugten ersten Teilschritt der Modifizierung der Festkörperoberfläche gemäß dem erfindungsgemäßen Verfahren (Teilschritt (aa)) werden funktionelle Gruppen auf der Festkörperoberfläche eingeführt. In einer bevorzugten Ausführungsform geschieht dies durch eine Aminofunktionalisierung, Epoxyfunktionalisierung, Halogenalkytfunktionalisierung und/oder Thiofunktionalisierung. Dies erfolgt dadurch, daß die Festkörperoberfläche mit entsprechend funktionellen Molekülen behandelt wird. Vorzugsweise werden hierfür die Moleküle, die die funktionellen Gruppen enthalten, in einer Lösung, beispielsweise in einer wässrigen Lösung, gelöst und das Oberflächenmaterial in diese Lösung gegeben bzw. die Lösung auf das Oberflächenmaterial aufgebracht. Die Inkubation kann beispielsweise bei Raumtemperatur über einige Stunden erfolgen. Je nach gewähltem Material können natürlich auch andere Reaktionsbedingungen gewählt werden. Weiterhin stellen die aufgezählten Funktionalitäten lediglich Beispiele dar, die von einem Fachmann um zusätzliche geeignete Möglichkeiten erweitert werden können.

In einer besonders bevorzugten Ausführungsform des erfindungsgemäßen Verfahrens werden für die Funktionalisierung Silane eingesetzt, die eine entsprechende Funktionalität tragen. Besonders geeignet sind hierfür mono-, di- oder trifunktionelle Silane. Es kann sich hierbei also folglich um aminofunktionelle, epoxyfunktionelle, halogenalkylfunktionelle und/oder thiofunktionelle Silane handeln.

Ein Beispiel für ein besonders geeignetes aminofunktionelles Silan ist N-(2-Aminoethyl)-3-Aminopropyltrimethoxysilan (EDA). Geeignete epoxyfunktionelle, halogenalkylfunktionelle bzw. thiofunktionelle Silane sind [3-(2,3-Epoxypropoxy)-propyl]trimethoxysilan (EPOXY), [3-lodopropyl]trimethoxysilan bzw. [3-Thiopropyl]trimethoxysilan.

In einer weiteren bevorzugten Ausführungsform werden für die Funktionalisierung Mercaptane und/oder Disulfide eingesetzt. Die Funktionalisierung mit Mercaptanen ist vor allem für metallische Festkörperoberflächen zu bevorzugen. Als Disulfide sind besonders Alkyldisulfide geeignet. Vorteilhafterweise tragen die Mercaptane und Disulfide funktionelle Gruppen.

Entsprechend wie bei den oben erwähnten Silanen können Mercaptane unterschiedlicher Funktionalität verwendet werden.

In einer besonders bevorzugten Ausführungsform wird als aminofunktionelles Mercaptan Cysteaminhydrochlorid und/oder Cysteamin eingesetzt. Selbstverständlich können auch andere Mercaptane, beispielsweise auch epoxyfunktionelle oder halogenalkylfunktionelle Mercaptane mit Erfolg eingesetzt werden.

Weiterhin können auch andere funktionelle Moleküle für eine Funktionalisierung der Festkörperoberfläche verwendet werden. Ein besonders geeignetes Beispiel hierfür ist Polyethylenimin (PEI), welches Aminofunktionen auf die Oberfläche bringt.

Als zweiter bevorzugter Teilschritt der Modifizierung der Festkörperoberfläche (Teilschritt (ab)) werden wechselwirkende Moleküle auf der funktionalisierten Oberfläche absorbiert. Diese Absorption an die funktionalisierte Oberfläche erfolgt insbesondere durch Wechselwirkung mit den funktionellen Gruppen, die in dem ersten Teilschritt auf die Oberfläche aufgebracht wurden. Es kann sich hierbei auch um eine sogenannte Chemisorption handeln, bei welcher kovalente Bindungen zwischen den wechselwirkenden Molekülen und den auf der Oberfläche angebrachten funktionellen Gruppen ausgebildet werden. Es kann sich jedoch auch um andere Wechselwirkungen zwischen den wechselwirkenden Molekülen und der funktionalisierten Oberfläche handeln, hierfür kommen beispielsweise elektrostatische Wechselwirkungen und auch van der Waalsche Kräfte in Frage.

In einer bevorzugten Ausführungsform des erfindungsgemäßen Verfahrens werden als wechselwirkende Moleküle Polymere eingesetzt. Dies können auch Biopolymere sein. Selbstverständlich können auch Monomere verwendet werden. Als Polymere sind besonders Polyelektrolyte, Polyampholyte und/oder Polyzwitterionen geeignet. Als Polyelektrolyte kommen vorzugsweise anionische Polyelektrolyte zum Einsatz. Zu den Polyampholyten zählen bekanntermaßen auch Proteine, die für diesen Teilschritt der Modifizierung der Festkörperoberfläche sehr geeignet sein können.

Einige Beispiele für Polyanionen bzw. anionische Polyelektrolyte, die besonders geeignet sind, sind Polysulfate, Polysulfonate, Polycarboxylate, Polyphosphate und deren freie Säuren, Polystyrolsulfonsäure, Polystyrolsulfonat (PSS), PAMAM-Dendrimere (Carboxylterminierte, halbzahlige Generation), Polyacrylsäure, Polyacrylat, Polymethacrylsäure, Polymethacrylat, Dextransulfat, Desoxyribonukleinsäure und Ribonukleinsäure. Beispiele für geeignete Polykationen sind Polyamine und deren Salze, Polyethylenimin, Polyallylamin, PAMAM-Dendrimere (Aminoterminierte, ganzzahlige Generation), Polyvinylamin, Polylysin, Poly(vinylpyridin) und deren Salze sowie Poly(diallyldimethylammoniumchlorid).

Ein Beispiel für ein geeignetes Protein, welches als wechselwirkendes Molekül erfindungsgemäß verwendet werden kann, ist Rinderserumalbumin (BSA). Es sind natürlich auch eine Vielzahl von weiteren Proteinen geeignet, insbesondere auch Enzyme.

In einer weiteren bevorzugten Ausführungsform der Erfindung werden als wechselwirkende (reaktive) Moleküle Substanzen eingesetzt, die mit der funktionalisierten Oberfläche kovalente Wechselwirkungen eingehen, wobei also eine sogenannte Chemisorption stattfindet. Besonders bevorzugt sind hierbei aminoreaktive Moleküle, die als wechselwirkende Moleküle für eine aminofunktionalisierte Oberfläche eingesetzt werden.

Besonders geeignete Beispiele hierfür sind die Polymere Poly(styrol-co-Maleinsäureanhydrid) (PSPMA) und Poly(ethylen-co-Maleinsäureanhydrid) (PEPMA). Weitere geeignete aminoreaktive Substanzen sind 3,3',4,4'-Benzophenontetracarbonsäuredianhydrid und 3,3',4,4'-Biphenyltetracarbonsäuredianhydrid.

Neben diesen aminoreaktiven Substanzen bzw. Polymeren werden weitere wechselwirkende bzw. reaktive Moleküle von der Erfindung mitumfasst, die sich einem Fachmann aus dem bisher Gesagten ohne weiteres erschließen.

In einer anderen bevorzugten Ausführungsform des erfindungsgemäßen Verfahrens wird die Oberfläche im ersten Teilschritt der Modifizierung mit epoxyfunktionellen Gruppen versehen, und in dem zweiten Teilschritt der Modifizierung mit entsprechend geeigneten epoxyreaktiven Molekülen behandelt. Beispiele für epoxyreaktive Polymere sind Polyethylenimin, Polyallylamin, Polyallylaminhydrochlorid, Polyvinylamin, Poly(ethylenglykol), Polyvinylalkohol und Dextran. Wie ersichtlich können Polyamine sowohl in einem einstufigen Prozess als Funktionalisierungsschicht, als auch in zweistufigen Prozessen als wechselwirkende Schicht aufgebracht werden. Dies ist immer dann von Vorteil, wenn in dem ersten Schritt die ursprünglichen Eigenschaften des Festkörpers durch eine kovalente Umsetzung der Oberflächenfunktionalitäten verändert werden sollen.

Auch für eine epoxyfunktionalisierte Oberfläche ist der Einsatz von Proteinen, insbesondere von Enzymen, als wechselwirkende Moleküle geeignet. Als weitere epoxyreaktive Substanzen kommen Natriumthiosulfat, Ethylendiaminhydrochlorid oder Taurin in Frage.

Durch den Einsatz von geladenen Molekülen, insbesondere Polymeren, bei der Modifizierung der Oberfläche können die Oberflächeneigenschaften dahingehend optimiert werden, daβ die dem Oberflächenmaterial eigenen unerwünschten Ladungseffekte minimiert werden. Eine entsprechende Auswahl der hierfür geeigneten Reagenzien ist je nach dem gewünschten Versuchsziel zu treffen.

In einer besonders bevorzugten Ausführungsform des erfindungsgemäßen Verfahrens werden zwischen der Festkörperoberfläche und der aufzubringenden Lipidschicht weitere Moleküle, die insbesondere eine weitere Funktionalität der Oberfläche bewirken, eingefügt. So ist es beispielsweise möglich, Farbstoffe, insbesondere Fluoreszenzfarbstoffe, Nanopartikel oder Biomoleküle, wie beispielsweise Proteine, insbesondere Enzyme, in die modifizierte Oberflächenschicht zu integrieren. Dies ermöglicht den Einbau von Sondenmolekülen in das System festkörperunterstützter Lipidschichten bzw. Lipidmembranen. Weiterhin ist es so möglich, chemisch oder photochemisch reaktive Funktionalitäten in die modifizierte Oberfläche einzubauen, was beispielsweise zur kovalenten Immobilisierung von transmembranen Proteinen in einer ansonsten fluiden, d.h. im wesentlichen flüssigen, Lipidmembran genutzt werden kann. Diese zusätzlich einzubringenden Funktionalitäten können beispielsweise kovalent oder auch über andere Wechselwirkungen mit der Festkörperoberfläche verbunden sein.
Als Festkörperoberflächen, also als Träger für die zu immobilisierende Lipidschicht oder Membran, kommen im Prinzip alle im Stand der Technik bekannten pulverförmigen Trägermaterialien in Frage. Vorteilhafterweise können diese Trägermaterialien auch porös sein, um eine noch größere Oberfläche zur Verfügung zu stellen.

Als Festkörperoberflächen sind insbesondere Silikatobertlächen bzw. Silikatpartikel geeignet. Weiterhin können poröse oder nicht-poröse Aluminate, Borate oder Zeolithe mit Erfolg eingesetzt werden. Außerdem geeignet sind kolloidale Lösungen von Edelmetallen, Metallen usw.. In einer besonders bevorzugten Ausführungsform werden als Festkörperoberflächen pulverförmige und vorzugsweise poröse Polymeroberflächen eingesetzt.

In einer weiteren bevorzugten Ausführungsform werden als Festkörperoberflächen magnetische Träger eingesetzt, beispielsweise Polymerkügelchen mit einem Magnetkern. Weiterhin können mit Vorteil Kern-Schalen-Polymerpartikel als Trägermaterial verwendet werden.

In einer bevorzugten Ausführungsform des erfindungsgemäßen Verfahrens werden solche Materialien als Festkörperoberflächenmaterial eingesetzt, die in chromatographischen Säulen als Packungsmaterial üblicherweise verwendet werden.

Darüberhinaus können als Oberflächenmaterialien Filme aus pulverförmigen Metallen, Halbleitern, Edelmetallen und/oder Polymeren eingesetzt werden, die auf insbesonders weitgehend planaren Trägermaterialien aufgebracht sind oder auf diesen aufgebracht werden können. Bei diesen Trägermaterialien kann es sich beispielsweise um Unterlagen aus Papier, Glass, Kunststoff oder dergleichen handeln, mit denen die Festkörper in geeigneter Weise verbunden werden, insbesondere durch verkleben oder verschmelzen.

Die Modifizierung der Festkörperoberfläche gemäß dem erfindungsgemäßen Verfahren macht die Oberfläche für die Immobilisierung von ganz unterschiedlichen Lipidschichten geeignet. Ganz besonders ist diese modifizierte Festkörperoberfläche für eine Immobilisierung von Lipiddoppelschichten, und insbesondere für eine Immobilisierung von Lipidmembranen geeignet. Lipiddoppelschichten bzw. Lipidmembranen sind auch von besonderem Interesse für die verschiedenartigen Anwendungen der Erfindung in der Forschung, Diagnostik und vor allem in der Biosensorik, wobei die nativen Eigenschaften solcher Schichten als Modelle für natürlichen Systeme genutzt werden können.

Erfindungsgemäß setzen sich die Lipidschichten aus Substanzen aus den Stoffklassen der Lipide, Lipidderivate, lipidähnlichen und/oder lipidanalogen Stoffe zusammen. Weiterhin können die Lipidschichten auch Peptide, Proteine, Nukleinsäuren, ionische oder nichtionische Tenside und/oder Polymere aufweisen. Durch diese weiteren Komponenten in den immobilisierten Lipidschichten können natürliche Systeme abgebildet werden, indem in den Lipidschichten beispielsweise Kanalproteine oder Enzyme enthalten sind. Diese zusätzlichen Komponenten können sich auf der Oberfläche der Lipidschicht oder eingebettet in der Lipidschicht, also integral, befinden. Weiterhin können sich die zusätzlichen Komponenten durch die ganze Schicht bzw. Membran hindurch, also transmembran (schichtdurchspannend), erstrecken. Bei diesen Proteinen kann es sich um Enzyme, Rezeptoren und/oder Liganden handeln, wobei sich die Rezeptoren und/oder Liganden vorzugsweise auf der dem Träger abgewandten Oberfläche der Lipidschicht zumindest teilweise orientieren. Weiterhin kann die Lipidmembran aus Proteoliposomen aufgebaut sein.

In einer besonders bevorzugten Ausführungsform des erfindungsgemäßen Verfahrens handelt es sich bei den zu immobilisierenden Lipidschichten zumindest teilweise um Membranfragmente aus natürlichen Zellen. Selbstverständlich können auch entsprechende künstliche Lipidschichten bzw. Membranen in vitro aus verschiedenen Komponenten zusammengefügt werden, so daß hierdurch ein entsprechendes Membranmodell zur Verfügung gestellt wird. In besonders bevorzugter Weise werden jedoch Membranen von natürlichen Zellen isoliert und auf den erfindungsgemäß modifizierten Oberflächen deponiert.

In einer weiteren Ausführungsform der Erfindung weist die immobilisierte Lipidschicht, insbesondere die Lipidmembran, Proteine auf, die vorzugsweise transmembran und/oder integral angeordnet sind, wobei diese Proteine wasserlösliche Proteine peripher binden können bzw. gebunden haben.

Das Deponieren der Lipidschichten auf der modifizierten Festkörperoberfläche geschieht durch herkömmliche Fusion von Lipidvesikeln. Bei diesen Vesikeln kann es sich um Vesikel definierter Zusammensetzung aus beispielsweise Phospholipiden handeln. Andererseits ist es natürlich auch möglich, Membranvesikel aus natürlichem Material zu isolieren und im Sinne der erfindungsgemäßen Erfindung zu verwenden. Besonders geeignet hierfür sind beispielsweise Membranvesikel, die aus dem sarkoplasmatischen Retikulum gewonnen werden. Die Bereitstellung der Vesikel erfolgt nach Methoden, die dem Fachmann bekannt sind. Ein großer Vorteil des erfindungsgemäßen Verfahrens ist darin zu sehen, daß derartige Vesikel lediglich mit der erfindungsgemäß modifizierten Oberfläche in Kontakt gebracht werden müssen, wodurch die Vesikel spontan miteinander verschmelzen und eine Lipidschicht ausbilden. Von daher handelt es sich bei dem erfindungsgemäßen Verfahren um ein ausgesprochen leicht zu handhabendes System, welches lediglich geringen präparativen Aufwand erfordert.

Die Erfindung umfasst ferner ein partikelförmiges Substrat mit einer hydrophil modifizierten Substratoberfläche sowie einer darauf immobilisierten Lipidschicht. Bezüglich der Merkmale der immobilisierten Lipidschicht wird auf die obige Beschreibung Bezug genommen.

Schließlich offenbart die vorliegende Anmeldung einen Kit zur Herstellung von immobilisierten Lipidschichten auf Festkörperoberflächen. Ein solcher Kit umfasst mindestens eine modifizierte Festkörperoberfläche gemäß der obigen Beschreibung. In einer anderen Variante umfasst der Kit Reagenzien zur Herstellung einer entsprechend modifizierten Festkörperoberfläche. Weiterhin kann es vorgesehen sein, daß der Kit zusätzlich Reagenzien zur Deponierung von Lipidschichten auf einer modifizierten Festkörperoberfläche aufweist. Es kann jedoch auch bevorzugt sein, daß diese Reagenzien von dem jeweiligen Anwender selbst bereitgestellt werden. Dies gilt vor allem dann, wenn es darum geht, Membranen aus natürlichen Systemen zu isolieren und diese erfindungsgemäß zu immobilisieren.

Die beschriebenen Merkmale und weitere Merkmale der Erfindung ergeben sich aus der folgenden Beschreibung von Beispielen in Verbindung mit den Figuren und den Unteransprüchen. Hierbei können die verschiedenen Merkmale jeweils für sich oder in Kombination miteinander verwirklicht sein.

In den Figuren ist das folgende dargestellt:
Figur 1 zeigt schematisch die Vorgehensweise beim erfindungsgemäßen Verfahren.
Figur 2 zeigt DSC-Meßkurven von mit Dielaidoylphosphatidylcholin (DEPC) beschichteten, porösen Silikatpartikeln mit und ohne optimierter Oberfläche im Vergleich mit natürlichen DEPC-Vesikeln ohne Trägermaterial.
Figur 3 zeigt Deuterium (²H)-NMR-Spektren von mit selektiv kettendeuteriertem Dipalmitoylphosphatidylcholin (DPPC)-d8 (7,7',8,8'-D₂) beschichteten, nichtporösen Silikatpartikeln mit und ohne optimierter Oberfläche im Vergleich mit natürlichen DPPC-d8 (7,7',8,8')-Vesikeln ohne Trägermaterial.
Figur 4 zeigt DSC-Messungen von DEPC-beschichteten, porösen Silikatpartikeln auf komplexen, optimierten Oberflächen.

### Beispiele

### 1. MODIFIZIERUNG DER FESTKÖRPEROBERFLÄCHE

### 1.1. FUNKTIONALISIERUNG DER FESTKÖRPEROBERFLÄCHE

### 1.1.1 Aminofunktionalisierung von pulverförmigen und porösen Sillkatoberflächen mit N-(2-Aminoethyl)-3-Aminopropyltrimethoxysilan (EDA)

Eine Silanlösung, bestehend aus 1,05 ml N-(2-Aminoethyl)-3-Aminopropyltrimethoxysilan (EDA) und 27 µl konzentrierter Essigsäure in 100 ml entionisiertem Wasser, wurde frisch hergestellt. Nach 5 Minuten wurden 5 g eines porösen Silikatmaterials (Nucleosil 4000-30 von Macherey-Nagel, Düren) zu der Silanlösung gegeben und unter Schütteln aufgeschlämmt. Diese Dispersion wurde drei Stunden langsam rotiert, danach wird das Silikatmaterial sedimentiert und dreimal mit entionisiertem Wasser gewaschen. Der Erfolg der Silanisierung wird mittels Infrarotspektroskopie in diffuser Reflexion (DRIFT) am getrockneten Silikatmaterial dokumentiert.

### 1.1.2. Aminofunktionalisierung von pulverförmigen und porösen Silikatoberflächen mit Polyethylenimin (PEI)

Zu einer Polyethylenimin (PEI)-Lösung bestehend aus 250 mg PEI (50% Lösung in Wasser, Aldrich, Steinheim) in 50 ml entionisiertem Wasser wurden 5 g eines porösen Silikatmaterials (Nucleosil 4000-10 von Macherey-Nagel, Düren) gegeben und drei Stunden langsam rotiert. Danach wurde das Silikatmaterial sedimentiert und dreimal mit entionisiertem Wasser gewaschen. Der Erfolg der Silanisierung wurde mittels Infrarotspektroskopie in diffuser Reflexion (DRIFT) am getrockneten Silikatmaterial dokumentiert.

### 1.1.3. Thiofunktionalisierung von pulverförmigen und porösen Silikatoberflächen

Eine Silanlösung, bestehend aus 1 ml Mercaptopropyl-trimethoxysilan (THIO) in 50 ml 2-Propanol, wurde frisch hergestellt. Nach 5 Minuten wurden 5 g eines porösen Silikatmaterials (Nucleosil 4000-30 von Macherey-Nagel, Düren) zu der Silanlösung gegeben und unter Schütteln aufgeschlämmt. Diese Dispersion wurde drei Stunden langsam rotiert, danach wurde das Silikatmaterial sedimentiert und der Überstand entfernt. Das Material wurde zunächst bei 80°C getrocknet und eine Stunde bei 100°C getempert. Danach wurde dreimal mit 2-Propanol gewaschen. Der Erfolg der Silanisierung wurde mittels DRIFT dokumentiert.

### 1.1.4. Epoxyfunktionalisierung von pulverförmigen und porösen Silikatoberflächen

Eine Silanlösung, bestehend aus 2 ml [3-(2,3-Epoxypropoxy)-propyl]trimethoxysilan (GPS) in 100 ml 2-Propanol wurde frisch hergestellt. Nach 5 Minuten wurden 10 g eines porösen Silikatmaterials (Nucleosil 4000-30 von Macherey-Nagel, Düren) zu der Silanlösung gegeben und unter Schütteln aufgeschlämmt. Diese Dispersion wurde drei Stunden langsam rotiert, danach wurde das Silikatmaterial sedimentiert und der Überstand entfernt. Das Material wurde zunächst bei 80°C getrocknet und eine Stunde bei 100°C getempert. Danach wurde dreimal mit 2-Propanol gewaschen. Der Erfolg der Silanisierung wird mittels DRIFT dokumentiert.

### 1.1.5. Aminofunktionalisierung von magnetischen Partikeln mit Polyethylenimin (PEI)

2 ml einer Suspension von magnetischen Polystyrolpartikeln (Dynabeads^{R} M-280, Tosylactivated, der Firma Dynal, Oslo, Norwegen) werden mit 0,1 M NaCl in Wasser gewaschen. Zu der entstehenden Suspension werden 5 ml einer Polyethylenimin (PEI)-Lösung bestehend aus 250 mg PEI (50% Lösung in Wasser, Aldrich, Steinheim) in 50 ml 0,1 M NaCl in entionisiertem Wasser gegeben und drei Stunden langsam rotiert. Danach werden die Partikel mit Hilfe eines Magneten abgetrennt und dreimal mit 0,1 M NaCl in Wasser gewaschen. Der Erfolg der Umsetzung wird mittels Titration bestimmt.

### 1.2. ADSORPTION/CHEMISORPTION VON WECHSELWIRKENDEN MOLEKÜLEN

### 1.2.1. Adsorption von Na-Polystyrotsulfonat (PSS) auf EDA-funktionalisierten Silikatoberflächen

Zu einer Na-Polystyrolsulfonat (PSS)-Lösung bestehend aus 12,5 mg PSS (Aldrich, Steinheim) in 25 ml entionisiertem Wasser wurde 1 g eines entsprechend Beispiel 1.1.1. aminofunktionalisiertem Silikatmaterial gegeben und drei Stunden geschüttelt. Danach wurde das Silikatmaterial sedimentiert und dreimal mit entionisiertem Wasser gewaschen. Der Erfolg der Adsorption wurde mittels DRIFT und der Abnahme der PSS-Konzentration in der Lösung dokumentiert.

### 1.2.2. Adsorption von Na-Polystyrolsulfonat (PSS) auf PEI-funktionalisierten Silikatoberflächen

Zu einer Na-Polystyrolsulfonat (PSS)-Lösung, bestehend aus 25 mg PSS (Aldrich, Steinheim) in 25 ml entionisiertem Wasser, wurde 1 g eines entsprechend Beispiel 1.1.2. aminofunktionalisiertem Silikatmaterials gegeben und drei Stunden geschüttelt. Danach wurde das Silikatmaterial sedimentiert und dreimal mit entionisiertem Wasser gewaschen. Der Erfolg der Adsorption wurde mittels DRIFT und der Abnahme der PSS-Konzentration in der Lösung dokumentiert.

### 1.2.3. Adsorption von Na-Polystyrolsulfonat (PSS) auf aminofunktionalisierten pulverförmigen Polymeroberflächen

Zu einer Lösung bestehend aus 3 ml 0,1 molarer Na-Polystyrolsulfonat (Aldrich, Steinheim)-Lösung sowie 5 ml einer 4,8 molaren NaCl-Lösung wurden 4 ml einer Latexdispersion, bestehend aus aminofunktionalisiertem Polystyrol-latices (K2-080, Prolabo, France), gegeben und über Nacht geschüttelt. Danach wurde das modifizierte Polymermaterial durch Zentrifugation abgetrennt. Zum Waschen wurde das abgesetzte Polymermaterial in 10 ml entionisiertem Wasser resuspendiert, geschüttelt und im Anschluß wieder zentrifugiert. Dieser Waschschritt wurde insgesamt viermal wiederholt und das Polymermaterial abschließend in 4 ml entionisiertem Wasser resuspendiert. Der Erfolg der Adsorption wurde durch Titration der Oberflächenladungen dokumentiert.

### 1.2.4. Chemisorption von Poly(styrol-co-maleinsäureanhydrid) (PSPMA) auf EDA-funktionalisierten Silikatoberflächen

Zu einer Poly(styrol-co-maleinsäureanhydrid) (PSPMA)-Lösung, bestehend aus 250 mg PSPMA (Aldrich, Steinheim) in 200 ml Aceton wurden 5 g eines entsprechend Beispiel 1.1.1. aminofunktionalisiertem Silikatmaterials gegeben und drei Stunden geschüttelt. Danach wurde das Silikatmaterial sedimentiert und dreimal mit Aceton gewaschen. Der Erfolg der Chemisorption wurde mittels DRIFT dokumentiert.

### 1.2.5. Chemisorption von Poly(styrol-co-maleinsäureanhydrid) (PSPMA) auf PEI-funktionalisierten Silikatoberflächen

Zu einer Poly(styrol-co-maleinsäureanhydrid) (PSPMA)-Lösung, bestehend aus 250 mg PSPMA (Aldrich, Steinheim) in 200 ml Aceton wurden 5 g eines entsprechend Beispiel 1.1.2. aminofunktionalisiertem Silikatmaterials gegeben und drei Stunden geschüttelt. Danach wurde das Silikatmaterial sedimentiert und dreimal mit Aceton gewaschen. Der Erfolg der Chemisorption wurde mittels DRIFT dokumentiert.

### 1.2.6. Adsorption von Rinderserumalbumin (BSA) auf EDA-funktionalisierten Silikatoberflächen

1 g eines entsprechend Beispiel 1.1.1. funktionalisierten EDA-Trägermaterials wurde in eine Lösung, bestehend aus 220 mg Rinderserumalbumin (BSA, von Sigma-Aldrich, Steinheim) in 50 ml eines 25 mM HEPES-Puffer pH 7,1 (Puffer A), gegeben und drei Stunden rotiert. Danach wurde das Trägermaterial sedimentiert, dreimal mit Puffer A gewaschen und anschließend getrocknet. Der Erfolg der Beschichtung wurde durch DRIFT und Messung der Abnahme der BSA-Konzentration in der Lösung dokumentiert.

### 1.2.7. Adsorption von Natriumthiosulfat auf epoxyfunktionalisierten Silikatoberflächen

Zur Herstellung einer anionischen Oberfläche wurde 1 g eines entsprechend Beispiel 1.1.4. hergestellten Materials für 16 h in einer 0,1 molaren Natriumthiosulfatlösung rotiert. Danach wurde das Material sedimentiert, dreimal mit entionisiertem Wasser gewaschen und bei 80°C getrocknet. Der Erfolg der Beschichtung wurde mittels DRIFT dokumentiert.

### 1.2.8. Chemisorption von Poylethylenimin auf epoxyfunktio nalisierte Silikatoberflächen

Zu einer Polyethylenimin (PEI)-Lösung bestehend aus 250 mg PEI (50% Lösung in Wasser, Aldrich, Steinheim) in 50 mL entionisiertem Wasser wurde 1 g eines entsprechend Beispiel 1.1.4. hergestellten Materials gegeben und drei Stunden langsam rotiert. Danach wurde das Material sedimentiert und dreimal mit entionisiertem Wasser gewaschen. Der Erfolg der Beschichtung wird mittels DRIFT dokumentiert.

### 1.2.9. Adsorption von Polyallylaminhydrochlorid (PAH) auf PSS-funktionalisierten Silikatoberflächen und weitere Adsorption von PSS

Zur Herstellung einer modifizierten Oberfläche in einem mehrstufigen Verfahren wurden zu einer Polyallylaminhydrochlorid (PAH)-Lösung, bestehend aus 12,5 mg PAH (Aldrich, Steinheim) in 25 ml entionisiertem Wasser, 1 g eines entsprechend Beispiel 1.2.1. PSS-funktionalisiertem Silikatmaterial gegeben und drei Stunden geschüttelt. Danach wurde das Silikatmaterial sedimentiert und dreimal mit entionisiertem Wasser gewaschen. Der Erfolg der Adsorption wurde mittels DRIFT dokumentiert. 0,5 g des erhaltenen PAH-funktionalisierten Silikatmaterials wurde in eine Lösung, bestehend aus 10 mg PSS (Aldrich, Steinheim) in 10 ml entionisiertem Wasser gegeben und drei Stunden geschüttelt. Danach wurde das Silikatmaterial sedimentiert und dreimal mit entionisiertem Wasser gewaschen. Der Erfolg der Adsorption wurde mittels DRIFT dokumentiert.

### 1.2.10. Adsorption von Na-Polystyrolsulfonat (PSS) auf PEI-funktionalisierte magnetische Partikeln

Zu der entsprechend Beispiel 1.1.5. hergestellten Suspension werden 5 ml einer Na-Polystyrolsulfonat (PSS)-Lösung bestehend aus 12,5 mg PSS (Aldrich, Steinheim) in 25 ml 0,1 M NaCl in entionisiertem Wasser gegeben und drei Stunden geschüttelt. Danach werden die Partikel abgetrennt und dreimal mit 0,1 M NaCl in entionisiertem Wasser gewaschen. Der Erfolg der Adsorption wird mittels Titration und der Abnahme der PSS-Konzentration dokumentiert.

### 1.3. EINFÜGUNG WEITERER FUNKTIONELLER MOLEKÜLE IN DIE MODIFIZIERTE OBERFLÄCHE

### 1.3.1. Herstellung photoreaktiver Oberflächen auf PSS/EDA-funktionalisierten Silikatoberflächen

1 g eines entsprechend Beispiel 1.2.1. funktionalisierten EDA/PSS-Trägermaterials wurde in eine Lösung, bestehend aus 0,2 g 3,3',4,4'-Benzophenontetracarbonsäuredianhydrid (BPA) in 25 ml Aceton, gegeben und über Nacht rotiert. Danach wurde das Trägermaterial sedimentiert, dreimal mit Aceton gewaschen und getrocknet. Der Erfolg der Behandlung wurde mittels DRIFT dokumentiert.

### 1.3.2. Herstellung photoreaktiver Oberflächen auf EDA-funktionalisierten Silikatoberflächen

1 g eines entsprechend Beispiel 1.1.1 funktionalisierten EDA-Trägermaterials wurde in eine Lösung, bestehend aus 0,2 g 3,3',4,4'-Benzophenontetracarbonsäuredianhydrid (BPA) in 25 ml Aceton, gegeben und über Nacht rotiert. Danach wurde das Trägermaterial sedimentiert, dreimal mit Aceton gewaschen und getrocknet. Der Erfolg der Behandlung wurde mittels DRIFT dokumentiert.

### 1.3.3. Herstellung von Anhydridoberflächen auf PSS/EDA-funktionalisierten Silikatoberflächen

1 g eines entsprechend Beispiel 1.2.1. funktionalisierten EDA/PSS-Trägermaterials wurde in eine Lösung, bestehend aus 0,1 g 3,3',4,4'-Biphenyltetracarbonsäuredianhydrid in 25 ml Aceton, gegeben und über Nacht rotiert. Danach wurde das Trägermaterial sedimentiert, dreimal mit Aceton gewaschen und getrocknet.

### 1.3.4. Herstellung von Farbstoffoberflächen auf PSS/EDA-funktionalisierten Silikatoberflächen

1 g eines entsprechend Beispiel 1.2.1. funktionalisierten EDA/PSS-Trägermaterials wurde in eine Lösung, bestehend aus 2 mg Fluoresceinisothiocyanat (FITC) in 10 ml Ethanol, gegeben und über Nacht rotiert. Danach wurde das Trägermaterial sedimentiert, dreimal mit Ethanol gewaschen und getrocknet.

### 1.4. LIPIDANKER

### 1.4.1. Herstellung von Lipidankern aus chemisch reaktiven Oberflächen

1 g eines entsprechend Beispiel 1.2.4. hergestellten EDA/PSPMA-Materials wurde für 16 Stunden in einer Lösung von 50 mg Dioleoylphosphatidylethanolamin (DOPE) in Triethylamin/Chloroform rotiert. Danach wurde das Material sedimentiert und dreimal mit Chloroform gewaschen. Der Erfolg der Beschichtung wurde mittels DRIFT dokumentiert.

### 1.4.2. Herstellung von Lipidankern aus ionischen Oberflächen

1 g eines entsprechend Beispiel 1.2.1. hergestellten EDA/PSS-Materials wurde für 16 Stunden in einer Lösung von 50 mg Dioctadecyldimethylammoniumbromid (DODAB) in Chloroform rotiert. Danach wurde das Material sedimentiert und dreimal mit Chloroform gewaschen. Der Erfolg der Beschichtung wurde mittels DRIFT dokumentiert.

### 2. DEPOSITION VON LIPIDSCHICHTEN AUF DEN MODIFIZIERTEN OBERFLÄCHEN

### 2.1. Herstellung von Lipidvesikel zur Immobilisierung auf pulverförmigen Oberflächen

80 mg Dielaidoylphosphatidylcholin (DEPC) wurden in 16 ml Beschichtungspuffer, bestehend aus 20 mM HEPES-Puffer pH 7,1 mit 30 mM NaCl, eine halbe Stunde bei Raumtemperatur gequollen und anschließend 30 Minuten mit einem Stabbeschaller (Branson Sonorex) ultrabeschallt. Das Ergebnis war eine klare Vesikeldispersion mit Vesikeldurchmessem im Bereich 20-80 nm. Die Bestimmung erfolgte mittels herkömmlicher dynamischer Laserlichtstreuung ("Particle-Sizing").

### 2.2. Immobilisierung von Lipidmembranen auf optimierten pulverförmigen Silikatobertlächen

Zu 16 ml einer entsprechend Beispiel 2.1 hergestellten Vesikeldispersion wurde 1 g eines entsprechend Beispiel 1.2.1. optimierten porösen Silikatträgers zugegeben und 30 Minuten langsam rotiert. Danach wurde das Trägermaterial sedimentiert und dreimal mit Beschichtungspuffer gewaschen. Der Erfolg der Beschichtung wurde mittels DSC an dem in Beschichtungspuffer dispergiertem Material (wie beschrieben bei C. Naumann, T. Brumm, T. M. Bayerl, Biophys. J., 1992, 63, 1314) und DRIFT (nach Trocknung des Materials) dokumentiert.

### 2.3. Immobilisierung von Lipidmembranen auf optimierten pulverförmigen Polymeroberflächen

Zu 4 ml einer entsprechend Beispiel 2.1 hergestellten Vesikeldispersion wurden 1,3 ml eines entsprechend Beispiel 1.2.3. modifizierten Polymerträgers zugegeben und 30 Minuten langsam rotiert. Danach wurde das Trägermaterial sedimentiert und dreimal mit Beschichtungspuffer gewaschen. Der Erfolg der Beschichtung wurde mittels DSC an dem in Beschichtungspuffer dispergierten Material dokumentiert.

### 2.4. Immobilisierung von Lipidmembranen auf optimierten pulverförmigen Oberflächen mit gemischter Funktionalität

Zu 16 ml einer entsprechend Beispiel 2.1 hergestellten Vesikeldispersion wurde 1 g eines entsprechend Beispiel 1.3.4. optimierten porösen Silikatträgers gegeben und 30 Minuten langsam rotiert. Danach wurde das Trägermaterial sedimentiert und dreimal mit Beschichtungspuffer gewaschen. Der Erfolg der Beschichtung wurde mittels DSC an dem in Beschichtungspuffer dispergiertem Material und DRIFT (nach Trocknung des Materials) dokumentiert.

### 2.5. Immobilisierung von Lipidmembranen auf optimierten pulverförmigen Oberflächen mit Lipidanker

Zu 16 ml einer entsprechend Beispiel 2.1 hergestellten Vesikeldispersion wurde 1 g eines entsprechend Beispiel 1.4.1. optimierten porösen Silikatträgers gegeben und 30 Minuten langsam rotiert. Danach wurde das Trägermaterial sedimentiert und dreimal mit Beschichtungspuffer gewaschen. Der Erfolg der Beschichtung wurde mittels DSC an dem in Beschichtungspuffer dispergiertem Material und DRIFT (nach Trocknung des Materials) dokumentiert.

### 2.6. Immobilisierung von nativen Sarkoplasmatischen Retikulum (SR)-Membranen auf EDA/PSS modifizierten Silikatoberflächen und Messung der Ca²⁺-ATPaseFunktion

Aus dem Muskelgewebe eines Kaninchens wurden nach einem Verfahren von W. Hasselbach und M. Makinose (Biochem Z. 1961, 333, 518-528) Membranvesikel des sarkoplasmatischen Retikulums hergestellt (SR-Vesikel). Diese Dispersion wurde anschließend durch Ultraschallbehandlung in kleine, einschalige Vesikel mit einem Durchmesser von 20-90 nm überführt. Zu 900 µl dieser Lösung (etwa 0,5 mg Gesamtprotein) wurden 50 mg eines entsprechend Beispiel 1.2.1. optimierten porösen Silikatträgers zugegeben und für 18 Stunden bei 4°C inkubiert, wobei als Pufferlösung 100 mM Triethanolamin (pH 7,4) und 100 mM NaCl (Inkubationspuffer) verwendet wurde. Danach wurde das Trägermaterial sedimentiert und dreimal mit Inkubationsspuffer gewaschen. Der Erfolg der Beschichtung wurde mittels DRIFT am getrockneten Material dokumentiert. Die Ca²⁺-ATPase-Aktivität auf dem Trägermaterial nach der Waschung im Inkubationspuffer erfolgte durch Bestimmung der ATP-Hydrolyse-Aktivität in Abhängigkeit der Calciumionenkonzentration und deren Hemmung durch den spezifischen Inhibitor "Cyclopiazonic Acid". Dieser Funktionstest belegte eine zum SR-Vesikel vergleichbare Ca²⁺-ATPase-Aktivität auf dem Trägermaterial.

### 2.7. Immobilisierung von nativen Sarkoplasmatischen Retikulum (SR)-Membranen auf GPS/PEI modifizierten Silikatoberflächen und Messung der Ca²⁺-ATPaseFunktion

Aus dem Muskelgewebe eines Kaninchens wurden nach einem Verfahren von W. Hasselbach und M. Makinose (Biochem Z. 1961, 333, 518-528) Membranvesikel des sarkoplasmatischen Retikulums hergestellt (SR-Vesikel). Diese Dispersion wurde anschließend durch Ultraschallbehandlung in kleine, einschalige Vesikel mit einem Durchmesser von 20-90 nm überführt. Zu 900 µl dieser Lösung (etwa 0,5 mg Gesamtprotein) wurden 50 mg eines entsprechend Beispiel 1.2.8. optimierten porösen Silikatträgers zugegeben und für 18 Stunden bei 4°C inkubiert, wobei als Pufferlösung 100 mM Triethanolamin (pH 7,4) und 100 mM NaCl (Inkubationspuffer) verwendet wurde. Danach wurde das Trägermaterial sedimentiert und dreimal mit Inkubationsspuffer gewaschen. Der Erfolg der Beschichtung wurde mittels DRIFT am getrockneten Material dokumentiert. Die Ca²⁺-ATPase-Aktivität auf dem Trägermaterial nach der Waschung im Inkubationspuffer erfolgte durch Bestimmung der ATP-Hydrolyse-Aktivität in Abhängigkeit der Calciumionenkonzentration und deren Hemmung durch den spezifischen Inhibitor "Cyclopiazonic Acid". Dieser Funktionstest belegte eine zum SR-Vesikel vergleichbare Ca²⁺-ATPase-Aktivität auf dem Trägermaterial.

### 3. EIGENSCHAFTEN DER MEMBRANEN AUF OPTIMIERTEM TRÄGERMATERIAL

### 3.1. Stabilität im fließenden wäßrigen Medium

Die in Beispielen 2.2. bis 2.6 beschriebenen Systeme wurden für die Dauer von 24 Stunden einem strömenden Medium (Beschichtungspuffer nach Beispiel 2.1 bzw. Inkubationspuffer nach Beispiel 2.6) in einem Testbad ausgesetzt. In Abständen von 2 Stunden wurden jeweils gleiche Mengen Trägermaterial aus dem Testbad entnommen, getrocknet und anschließend mittels DRIFT auf ihre Beschichtung untersucht. Die in den Beispielen 2.2 bis 2.5 beschriebenen Systeme wurden zusätzlich mittels DSC untersucht. Weder mit DRIFT noch mit DSC wurde eine meßbare Abnahme der Membranbeschichtung mit der Zeit beobachtet.

### 3.2. Stabilität nach Trocknung und erneuter Dispersion

Die in den Beispielen 2.2 bis 2.5 beschriebenen Systeme wurden getrocknet und die auf ihnen befindlichen Lipidmenge mittels DRIFT dokumentiert. Anschließend wurden die Proben im Beschichtungspuffer resuspendiert und dreimal gewaschen. Nach dieser Behandlung wurde die Probe erneut getrocknet und mittels DRIFT vermessen. In allen Fällen war keine Veränderung der auf dem Trägermaterial befindlichen Lipidmenge nachweisbar.

### 3.3. Stabilität nach Einfrieren

Die in den Beispielen 2.2 bis 2.5 beschriebenen Systeme wurden bei -80°C im dispergierten Zustand eingefroren und anschließend wieder auf Raumtemperatur gebracht und getrocknet. Vergleichende DRIFT-Messungen vor und nach dem Einfrieren ergaben unveränderte Lipidmengen auf dem Trägermaterial.

### 3.4. Stabilität der enzymatischen Aktivität von SR-beschichteten Trägermaterial

Das in Beispiel 2.6 beschriebene System wurde nach der Präparation über die Dauer von 3 Monaten bei -80°C aufbewahrt. Im Abstand von 1 Monat wurden Proben entnommen und auf ihre Ca²⁺-ATPase-Aktivität mittels des in 2.6 beschriebenen Verfahrens untersucht. Nach 2 Monaten war die Aktivität auf ca. 70% des ursprünglichen Wertes (unmittelbar nach der Präparation und Waschung des Trägermaterials gemessen) abgesunken. Im Überstand der gelagerten Proben konnte keine Ca²⁺-ATPase-Aktivität gemessen werden.

### 3.5. Vergleich der Phasenübergangstemperaturen

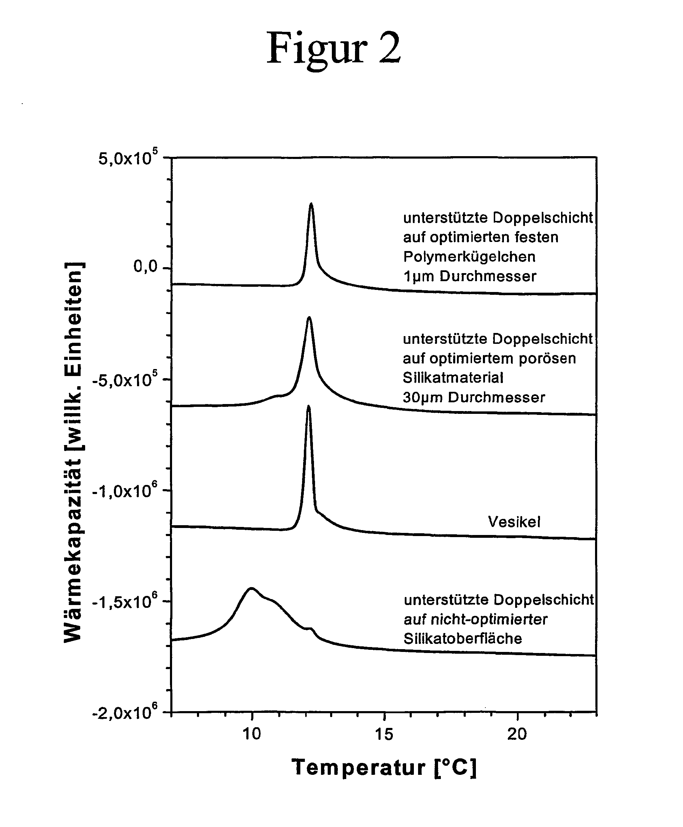

Figur 2 zeigt vergleichende differentialkalorimetrische (DSC)-Messungen des Phasenübergangs festkörperunterstützter Bilayer, bestehend aus dem synthetischen Lipid Dielaidoyl-sn-3-glycero-3-phosphocholin (im folgenden DEPC genannt) auf einer nichtoptimierten Festkörperoberfläche (Herstellung nach Stand der Technik, z.B. C. Naumann, T. Brumm, T. M. Bayerl, Biophys. J., 1992, 63, 1314), auf einer durch den obigen Schritt optimierten Oberfläche (wie im Beispiel beschrieben) im Vergleich zum natürlichen Analogon, den DEPC-Vesikeln ohne Festkörperunterstützung (Herstellung nach Stand der Technik durch Quellen des Lipids und anschließende Extrusion gemäß M. J. Hope, M. B. Bally, G. Web, P.R. Cullis, Biochim. Biophys. Acta 1985, 812, 55).

Diese Ergebnisse zeigen eine deutliche Verschiebung und Verbreiterung des Phasenübergangs bei der nichtoptimierten Oberfläche im Vergleich zu den (festkörperfreien) DEPC-Vesikeln. Die Phasenübergangstemperatur auf der optimierten Oberfläche wird nicht beeinflußt. Der Phasenübergang selbst ist im Vergleich zum natürlichen Analogon nur unwesentlich verbreitert.

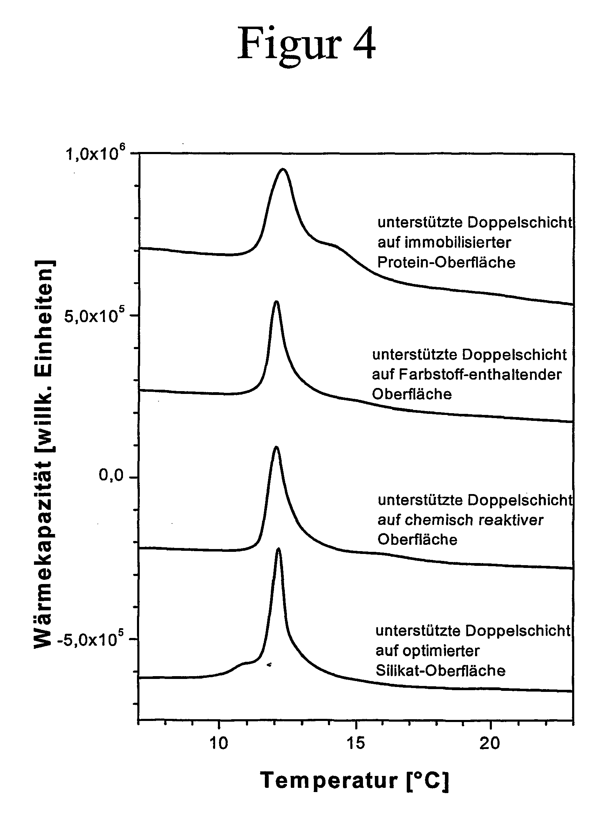

Figur 4 zeigt DSC-Messungen von DEPC-Lipidmembranen auf verschiedenen komplexen Oberflächen. Dabei zeigen alle dargestellten Beispiele nahezu keine Verschiebung der Phasenübergangstemperatur und nur eine unwesentliche Verbreiterung des Phasenüberganges selbst.

### 3.6. Vergleichende Deuterium (²H)-NMR-Messungen

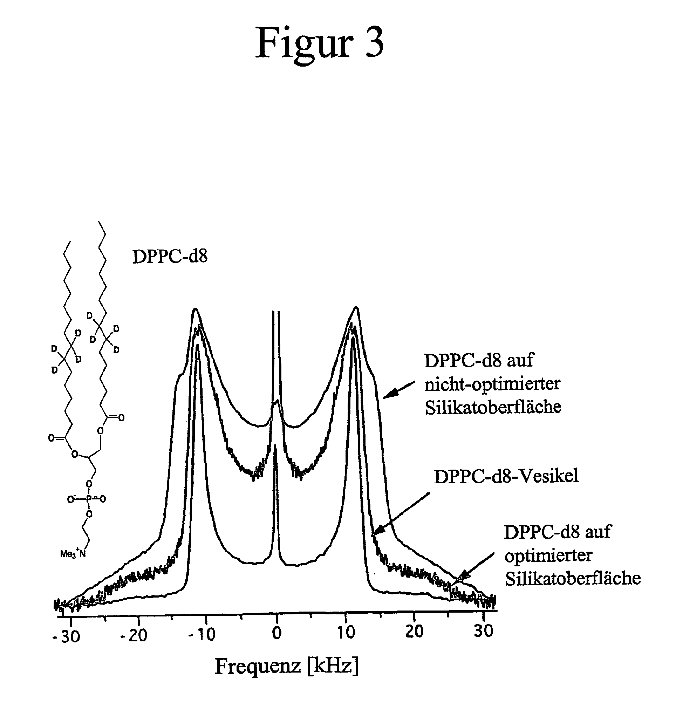

Figur 3 zeigt Deuterium (²H)-NMR-Messungen von kettendeuteriertem Dipalmitoylphosphatidylcholin (DPPC)-d8 (7,7',8,8') beschichteten nichtporösen Silikatpartikeln mit und ohne optimierter Oberfläche im Vergleich mit natürlichen DPPC-d8 (7,7',8,8')-Vesikeln. Die Quadrupolaufspaltung des von den selektiv deuterierten Lipidketten herrührenden Signals ist ein Maß für die molekulare Ordnung im Bilayer (Seelig, J. Quarterly Reviews of Biophysics 1977, 10, 353-418). Für den Fall der nichtmodifizierten Oberfläche resultieren zwei Aufspaltungen, die bereits früher mit der durch die unmittelbare Nähe der Festkörperoberfläche verursachten Asymmetrie der molekularen Ordnung in der inneren und äußeren Monolage des Bilayers erklärt wurde (M. Hetzer, S. Heinz, S. Grage, T. M. Bayerl, Langmuir, 1998, 14, 982-984). Die zweifache Aufspaltung findet sich weder bei dem optimierten System noch bei dem natürlichen Analogon (Vesikel) und ist deshalb als Beweis einer Äquivalenz der molekularen Ordnung in beiden Monolagen des Bilayers anzusehen.

## Patentansprüche

1. Verfahren zum Immobilisieren von Lipiddoppelschichten auf Oberflächen partikelförmiger Substrate, so dass die Substratoberfläche von der Lipiddoppelschicht vollständig umschlossen wird, wobei die Substratoberflächen derart modifiziert werden, dass die darauf deponierten Lipiddoppelschichten in ihren Eigenschaften denen nicht-immobilisierter Lipiddoppelschichten weitgehend entsprechen, **gekennzeichnet durch** die Verfahrensschritte
a) Modifizieren der Substratoberfläche mit Molekülen zur Ausbildung einer hydrophilen Fläche und
b) Deponieren der Lipiddoppelschichten auf der modifizierten Substratoberfläche,
wobei das Deponieren der Lipiddoppelschichten auf der modifizierten Substratoberfläche **durch** Fusion von Lipidvesikeln vorgenommen wird.

2. Verfahren nach Anspruch 1, **dadurch gekennzeichnet, dass** das Modifizieren der Substratoberfläche die folgenden Teilschritte umfasst:
aa) Funktionalisieren der Substratoberfläche und/oder
ab) Adsorption oder Chemisorption von wechselwirkenden Molekülen.

3. Verfahren nach Anspruch 1 oder Anspruch 2, **dadurch gekennzeichnet, dass** das Modifizieren der Substratoberfläche die folgenden Teilschritte umfasst:
aa) Funktionalisieren der Substratoberfläche und/oder
ab) Adsorption oder Chemisorption von wechselwirkenden Molekülen und
ac) Adsorption oder Chemisorption weiterer wechselwirkender Moleküle.

4. Verfahren nach Anspruch 2 oder Anspruch 3, **dadurch gekennzeichnet, dass** das Funktionalisieren der Substratoberfläche durch Aufbringen von Aminofunktionen, Epoxyfunktionen, Halogenalkylfunktionen und/oder Thiofunktionen vorgenommen wird, wobei insbesondere die Substratoberfläche mit aminofunktionellen, epoxyfunktionellen, halogenalkylfunktionellen und/oder thiofunktionellen Molekülen behandelt wird.

5. Verfahren nach einem der Ansprüche 2 bis 4, **dadurch gekennzeichnet, dass** für die Funktionalisierung Silane eingesetzt werden.

6. Verfahren nach einem der Ansprüche 2 bis 5, **dadurch gekennzeichnet, dass** für die Funktionalisierung Mercaptane und/oder Disulfide, insbesondere Alkyldisulfide, eingesetzt werden.

7. Verfahren nach einem der Ansprüche 2 bis 6, **dadurch gekennzeichnet, dass** für die Funktionalisierung N-(2-Aminoethyl)-3-Aminopropyltrimethoxysilan (EDA), Polyethylenimin (PEI) und/oder Cysteamin, insbesondere Cysteaminhydrochlorid, eingesetzt wird.

8. Verfahren nach einem der Ansprüche 2 bis 7, **dadurch gekennzeichnet, dass** als wechselwirkende Moleküle Polymere, insbesondere Polyelektrolyte, vorzugsweise anionische Polyelektrolyte, Polyampholyte, vorzugsweise Proteine, und/oder Polyzwitterionen eingesetzt werden.

9. Verfahren nach einem Ansprüche 2 bis 8, **dadurch gekennzeichnet, dass** als wechselwirkende Moleküle Polystyrolsulfonat (PSS), insbesondere Na-Polystyrolsulfonat, und/oder Poly(styrol-co-Maleinsaureanhydrid) (PSPMA) eingesetzt werden.

10. Verfahren nach einem der vorhergehenden Ansprüche, **dadurch gekennzeichnet, dass** bei dem Modifizieren der Substratoberfläche weitere Moleküle, insbesondere funktionelle Moleküle, in die Oberfläche eingefügt werden.

11. Verfahren nach Anspruch 10, **dadurch gekennzeichnet, dass** es sich bei den funktionellen Molekülen um Farbstoffe, insbesondere Fluoreszenzfarbstoffe, und/oder um enzymatisch, chemisch und/oder photochemisch reaktive Moleküle handelt.

12. Verfahren nach einem der vorhergehenden Ansprüche, **dadurch gekennzeichnet, dass** es sich bei den Substraten um im wesentlichen poröse, pulverförmige Substrate handelt.

13. Verfahren nach einem der vorhergehenden Ansprüche, **dadurch gekennzeichnet, dass** es sich bei den Substraten um pulverförmige Silikate und/oder pulverförmige Polymere handelt.

14. Verfahren nach einem der vorhergehenden Ansprüche, **dadurch gekennzeichnet, dass** es sich bei den Substraten um pulverförmige magnetische Partikel und/oder Kern-Schalen-Polymerpartikel handelt.

15. Verfahren nach einem der vorhergehenden Ansprüche, **dadurch gekennzeichnet, dass** die Substrate auf einem im wesentlichen planaren Träger aufgebracht sind oder auf diesen aufgebracht werden.

16. Verfahren nach einem der vorhergehenden Ansprüche, **dadurch gekennzeichnet, dass** die Substrate, insbesondere bei der Modifikation ihrer Oberfläche, in Form einer Dispersion, vorzugsweise in Form einer Dispersion in Wasser und/oder Alkohol, eingesetzt werden.

17. Verfahren nach einem der vorhergehenden Ansprüche, **dadurch gekennzeichnet, dass** es sich bei der Substratoberfläche um eine Silikatoberfläche, Halbleiteroberfläche, insbesondere Siliziumoberfläche, Edelmetalloberfläche, insbesondere Goldoberfläche, und/oder Polymeroberfläche, insbesondere Polystyrol-Oberfläche handelt.

18. Verfahren nach einem der vorhergehenden Ansprüche, **dadurch gekennzeichnet, dass** die Lipiddoppelschichten Lipidmembranen sind.

19. Verfahren nach einem der vorhergehenden Ansprüche, **dadurch gekennzeichnet, dass** die Lipiddoppelschichten aus Lipiden, Lipidderivaten, lipidähnlichen und/oder lipidanalogen Substanzen aufgebaut sind.

20. Verfahren nach einem der vorhergehenden Ansprüche, **dadurch gekennzeichnet, dass** die Lipiddoppeischichten weiterhin Peptide, Proteine, Nukleinsauren, ionische oder nichtionische Tenside und/oder Polymere aufweisen.

21. Verfahren nach einem der vorhergehenden Ansprüche, **dadurch gekennzeichnet, dass** die Lipiddoppelschichten oberflächlich, schichtdurchspannend und/oder darin eingebettet Proteine, insbesondere Enzyme, Rezeptoren und/oder Liganden aufweisen.

22. Verfahren nach einem der vorhergehenden Ansprüche, **dadurch gekennzeichnet, dass** die Lipiddoppelschichten zumindest teilweise aus Membranfragmenten von natürlichen Zellen aufgebaut sind.

23. Partikelförmiges Substrat mit einer hydrophil modifizierten Substratoberfläche und einer darauf immobilisierten Lipiddoppelschicht, wobei die Substratoberfläche von der Lipiddoppelschicht vollständig umschlossen wird, herstellbar durch ein Verfahren gemäss mindestens einem der Ansprüche 1 bis 22.

## Claims

1. A method for immobilizing double lipid layers on surfaces of particulate substrates, in a manner that the substrate surface is completely surrounded by a double lipid layer, modifying the substrate surfaces such that the double lipid layers which have been deposited on them, as regards their characteristics, to a large extent correspond to those of lipid layers which have not been immobilized, the method being **characterized by** the following procedural steps:
a) modifying the substrate surface using molecules in order to form an hydrophilic area, and
b) depositing the lipid double layers on the modified substrate surface, wherein the lipid double layers are deposited on the modified substrate surface by fusing of lipid vesicles.

2. The method according to claim 1, **characterized in that** the modifying of the substrate surface comprises the following substeps:
aa) functionalizing the substrate surface and/or
ab) adsorbing or chemisorbing interacting molecules.

3. The method according to claim 1 or claim 2, **characterized in that** the modifying of the substrate surface comprises the following substeps:
aa) functionalizing the substrate surface and/or
ab) adsorbing or chemisorbing interacting molecules, and
ac) adsorbing or chemisorbing additional interacting molecules.

4. The method according to claim 2 or claim 3, **characterized in that** the substrate surface is functionalized by applying amino functions, epoxy functions, haloalkyl functions and/or thio functions, in particular, with the substrate surface being treated with amino functional, epoxy functional, haloalkyl functional and/or thio functional molecules.

5. The method according to any of the claims 2 to 4, **characterized in that** silanes are used for the functionalization.

6. The method according to any of the claims 2 to 5, **characterized in that** mercaptanes and/or disulfides, in particular alkyl disulfides, are used for the functionalization.

7. The method according to any of the claims 2 to 6, **characterized in that** N-(2-aminoethyl)-3-aminopropyl-trimethoxy silane (EDA), polyethyleneimine (PEI) and/or cysteamine, in particular cysteamine hydrochloride, is/are used for the functionalization.

8. The method according to any of the claims 2 to 7, **characterized in that** polymers, in particular polyelectrolytes, preferably anionic polyelectrolytes, polyampholytes, preferably proteins, and/or polyzwitterions are used as interacting molecules.

9. The method according to any of the claims 2 to 8, **characterized in that** polystyrenesulfonate (PSS), in particular Na-polystyrenesulfonate, and/or poly(styrene-co-maleic anhydride) (PSPMA) is/are used as interacting molecule(s).

10. The method according to any of the preceding claims, **characterized in that** in connection with modifying the substrate surface, further molecules, in particular functional molecules, are inserted into the surface.

11. The method according to claim 10, **characterized in that** the functional molecules are dyes, in particular fluorescent dyes, and/or enzymically, chemically and/or photochemically reactive molecules.

12. The method according to any of the preceding claims, **characterized in that** the substrates are essentially porous powder substrates.

13. The method according to any of the preceding claims, **characterized in that** the substrates are powder silicates and/or powder polymers.

14. The method according to any of the preceding claims, **characterized in that** the substrates are powder magnetic particles and/or core-shell polymer particles.

15. The method according to any of the preceding claims, **characterized in that** the substrates have been applied to an essentially planar support or are applied to this support.

16. The method according to any of the preceding claims, **characterized in that** the substrates, in particular in connection with the modification of their surface, are used in the form of a dispersion, preferably in the form of a dispersion in water and/or alcohol.

17. The method according to any of the preceding claims, **characterized in that** the substrate surface is a silicate surface, a semiconductor surface, in particular a silicon surface, a precious metal surface, in particular a gold surface, and/or a polymer surface, in particular a polystyrene surface.

18. The method according to any of the preceding claims, **characterized in that** the double lipid layers are lipid membranes.

19. The method according to any of the preceding claims, **characterized in that** the double lipid layers are composed of lipids, lipid derivatives, lipid-like substances and/or lipid-analogous substances.

20. The method according to any of the preceding claims, **characterized in that** the double lipid layers additionally contain peptides, proteins, nucleic acids, ionic or non-ionic surfactants and/or polymers.

21. The method according to any of the preceding claims, **characterized in that** the double lipid layers contain proteins, in particular enzymes, receptors and/or ligands, which are present supeficially, such that they span the layer and/or such that they are embedded in it.

22. The method according to any of the preceding claims, **characterized in that** the double lipid layers are at least partially composed of membrane fragments derived from natural cells.

23. A particulate substrate, having a substrate surface modified to be hydrophilic, and a double lipid layer immobilized thereon, the substrate surface being completely surrounded by the double lipid layer, wherein the substrate may be prepared using a method according to any of the claims 1 to 22.

## Revendications

1. Procédé pour l'immobilisation de doubles couches de lipides sur des surfaces de substrats particulaires, de manière telle que la surface du substrat est complètement entourée par la double couche de lipides, les surfaces du substrat étant modifiées de manière telle que les doubles couches de lipides qui y ont été déposées correspondent dans une large mesure, en ce qui concerne leurs propriétés, à celles des doubles couches de lipides non immobilisées, **caractérisé par** les étapes de procédé
a) modification de la surface du substrat par des molécules avec formation d'une surface hydrophile et
b) dépôt des doubles couches de lipides sur la surface modifiée du substrat,
le dépôt des doubles couches de lipides sur la surface modifiée du substrat étant réalisé par fusion de vésicules lipidiques.

2. Procédé selon la revendication 1, **caractérisé en ce que** la modification de la surface du substrat comprend les étapes partielles suivantes :
aa) fonctionnalisation de la surface du substrat et/ou
ab) adsorption ou chimisorption de molécules interactives.

3. Procédé selon la revendication 1 ou la revendication 2, **caractérisé en ce que** la modification de la surface du substrat comprend les étapes partielles suivantes:
aa) fonctionnalisation de la surface du substrat et/ou
ab) adsorption ou chimisorption de molécules interactives et
ac) adsorption ou chimisorption d'autres molécules interactives.

4. Procédé selon la revendication 2 ou la revendication 3, **caractérisé en ce que** la fonctionnalisation de la surface du substrat est réalisée par application de fonctions amino, époxy, halogénoalkyle et/ou thio, la surface du substrat étant en particulier traitée par des molécules à fonctionnalité amino, époxy, halogénoalkyle et/ou thio.

5. Procédé selon l'une quelconque des revendications 2 à 4, **caractérisé en ce qu'**on utilise des silanes pour la fonctionnalisation.

6. Procédé selon l'une quelconque des revendications 2 à 5, **caractérisé en ce qu'**on utilise des mercaptans et/ou des disulfures, en particulier des alkyldisulfures, pour la fonctionnalisation.

7. Procédé selon l'une quelconque des revendications 2 à 6, **caractérisé en ce qu'**on utilise, pour la fonctionnalisation, du N-(2-aminoéthyl)-3-aminopropyltriméthoxysilane (EDA), de la polyéthylèneimine (PEI) et/ou de la cystéamine, en particulier le chlorhydrate de cystéamine.

8. Procédé selon l'une quelconque des revendications 2 à 7, **caractérisé en ce qu'**on utilise comme molécules interactives des polymères, en particulier des polyélectrolytes, de préférence des polyélectrolytes anioniques, des polyampholytes, de préférence des protéines et/ou des polyzwittérions.

9. Procédé selon l'une quelconque des revendications 2 à 8, **caractérisé en ce qu'**on utilise comme molécules interactives du polystyrènesulfonate (PSS), en particulier le polystyrènesulfonate de Na, et/ou le poly(styrène-co-anhydride de l'acide maléique) (PSPMA).

10. Procédé selon l'une quelconque des revendications précédentes, **caractérisé en ce qu'**on introduit, lors de la modification de la surface du substrat, d'autres molécules, en particulier des molécules fonctionnelles, dans la surface.

11. Procédé selon la revendication 10, **caractérisé en ce qu'**il s'agit, pour les molécules fonctionnelles, de colorants, en particulier de colorants de fluorescence, et/ou de molécules enzymatiquement, chimiquement et/ou photochimiquement réactives.

12. Procédé selon l'une quelconque des revendications précédentes, **caractérisé en ce qu'**il s'agit, pour les substrats, de substrats essentiellement poreux, poudreux.

13. Procédé selon l'une quelconque des revendications précédentes, **caractérisé en ce qu'**il s'agit, pour les substrats, de silicates poudreux et/ou de polymères poudreux.

14. Procédé selon l'une quelconque des revendications précédentes, **caractérisé en ce qu'**il s'agit, pour les substrats, de particules magnétiques poudreuses et/ou de particules polymères à noyau-coquille poudreuses.

15. Procédé selon l'une quelconque des revendications précédentes, **caractérisé en ce que** les substrats ont été appliqués ou sont appliqués sur un support essentiellement plat.

16. Procédé selon l'une quelconque des revendications précédentes, **caractérisé en ce que** les substrats, en particulier lors de la modification de leur surface, sont utilisés sous forme d'une dispersion, de préférence sous forme d'une dispersion dans l'eau et/ou un alcool.

17. Procédé selon l'une quelconque des revendications précédentes, **caractérisé en ce qu'**il s'agit, pour la surface du substrat, d'une surface d'un silicate, d'une surface semi-conductrice, en particulier d'une surface de silicium, d'une surface de métal noble, en particulier d'une surface d'or, et/ou d'une surface polymère, en particulier d'une surface de polystyrène.

18. Procédé selon l'une quelconque des revendications précédentes, **caractérisé en ce que** les doubles couches de lipides sont des membranes de lipides.

19. Procédé selon l'une quelconque des revendications précédentes, **caractérisé en ce que** les doubles couches de lipides sont formées de lipides, de dérivés de lipides, de substances semblables à des lipides et/ou analogues à des lipides.

20. Procédé selon l'une quelconque des revendications précédentes, **caractérisé en ce que** les doubles couches de lipides présentent en outre des peptides, des protéines, des acides nucléiques, des agents tensioactifs ioniques ou non ioniques et/ou des polymères.

21. Procédé selon l'une quelconque des revendications précédentes, **caractérisé en ce que** les doubles couches de lipides présentent des protéines, en particulier des enzymes, des récepteurs et/ou des ligands, en surface, à travers la surface et/ou enrobés dans celles-ci.

22. Procédé selon l'une quelconque des revendications précédentes, **caractérisé en ce que** les doubles couches de lipides sont au moins partiellement formées de fragments membranaires de cellules naturelles.

23. Substrat particulaire présentant une surface de substrat modifiée de manière hydrophile et une double couche de lipides qui y est immobilisée, la surface du substrat étant complètement entourée par la double couche de lipides, ledit substrat pouvant être préparé par un procédé selon au moins l'une quelconque des revendications 1 à 22.
